# EUROPEAN PATENT SPECIFICATION

(11) **EP 0 428 009 B1**
(45) Date of publication and mention of the grant of the patent: **05.10.1994**
(21) Application number: 90120851.2
(22) Date of filing: 31.10.1990
(51) Int. Cl.: A61M 1/14, A61L 2/04

(54) **A method for the preparation of a sterile dialysis fluid for medical use or a similar physiologically acceptable sterile fluid**
Verfahren zum Herstellen einer sterilen Flüssigkeit für medizinischen Gebrauch oder einer ähnlichen physiologisch akzeptablen sterilen Flüssigkeit
Méthode de préparation d'un fluide de dialyse stérile à usage médical ou d'un fluide stérile similaire physiologiquement compatible

(30) Priority: 16.11.1989 SE 8903855
(43) Date of publication of application: 22.05.1991
(73) Proprietor: GAMBRO AB, S-220 10 Lund (SE)
(72) Inventor: Lindqvist, Sten-Börje, S-240 14 Veberöd (SE); Nystrand, Rolf, S-222 47 Lund (SE)
(74) Representative: Asketorp, Göran

(56) References cited:
- FR-A- 2 073 337
- FR-A- 2 627 479
- GB-A- 1 504 334

## Description

### TECHNICAL FIELD

The present invention relates to a method for the preparation of a sterile dialysis fluid for medical use or a similar physiologically acceptable sterile fluid by which method water is continuously transported from a source for the same to a point of consumption, necessary concentrates in liquid and/or powder form being added during the transport, the water with our without concentrate being heated during the transport to sterilizing temperature without formation of steam and being kept at this temperature during the time required for the sterilization, whereafter it is cooled to the desired consuming temperature.

The invention also relates to a system for the preparation of a sterile dialysis fluid or a similar physiologically acceptable fluid by means of the above method, comprising a pipeline between said source for the water and the point of consumption, e g a draw-off point, this pipeline comprising means for the pumping of water, means for its heating, means for its cooling and means for the supply of necessary concentrates in liquid and/or powder form, the pipeline between the said means for heating and cooling respectively of the water being dimensioned so in relation to the capacity of the said means for pumping and heating respectively that the sojourn time of the water within this part of the pipeline will be sufficient for the sterilization.

The invention is this intended primarily to be employed in connection with preparation of a dialysis fluid, but can also be used for the preparation of other sterile fluids, e g wound cleansing fluid.

### BACKGROUND ART

The manufacture of sterile solutions and infusion solutions is regulated in national and international legislation with regard to the demands to be made in respect of chemical and microbiological purity. The method of sterilization generally used is steam autoclaving, that is to say sterilization with saturated water vapour at high temperature. A customary choice of parameters is 121°C for 15 minutes.

The fluid which is sterilized may be e g a physiological salt solution packed in some kind of container which usually consists of plastics or glass.

The disadvantage of the above mentioned system is obvious. As the refining value is low, the cost of handling and transport signify a great deal for the final cost of the product.

The present invention is based on the principle of producing the sterile solution directly at the place of application. The field of application here is very wide. It may be a matter of solutions which are used in dialysis, e g sterile CAPD solution or sterile hemofiltration solution or dialysis solution for conventional hemodialysis. Other fields of application are e g sterile wound cleansing solutions and sterile infusion solutions of different types. The above mentioned solutions may be produced in that water of suitable chemical and microbiological quality is prepared and is sterilized subsequently as the type of consumption necessitates. In accordance with the invention it is intended for this to take place continuously in a pipe system at the location of the actual utilization.

To check the resulting sterilization spores of Bacillus stearothermophilus bacteria may be used which, owing to their resistance, are a generally accepted indicator organism in steam sterilization.

The D-value is defined as the time it take to kill a ¹⁰logarithm of a given quantity of the spore form of Bacillus stearothermophilus at a given temperature. The lowest accepted D-value at 120°C for Bacillus stearothermophilus is 1.5 minutes. If the temperature is raised by 10°C the D-value generally drops to one tenth. This means that 130°C furnishes a D-value of the order of magnitude of 0.15 minutes.

The concept "sterile" implies free from living microorganisms. This requirement is converted in practice to a statistic definition. This means that on 1 000 000 units produced there may be not more than 1 unit with 1 surviving microorganism. This final result can be achieved by two methods, on the one hand by the overkilling concept, which implies that the sojourn time during sterilization should correspond to the killing of 12 D-values (10¹² spores of Bacillus stearothermophilus) or a concept which is based on the true number of heat-resistant microorganisms which are present in the solution prior to the sterilization. This concept makes it necessary, therefor, for the said number to be known. At the same time it normally means that a smaller number of D-values can be used. In general it is customary for the sojourn time to be adjusted to 8-10 D-values.

The closest prior art may be said to be GB-A-1 504 334. The system described is, however, difficult to sterilize by heating due to to the fact that it includes details which can not stand a temperature high enough for sterilization.

Please compare also the two French publications FR-A-2 073 337 and FR-A-2 627 479. The first one discloses a dialysis system in which the fluid may be recirculated for sterilization. This system can not, however, be used in practice due to the fact that the fluid is recirculated in such a way that the fluid path in front of the dialyzer most probably will be contaminated with contaminations removed from the fluid path downstream of the dialyzer. The second one discloses a system for the production of especially "eau de ville". Consequently, you can not talk about a real sterilization.

### DISCLOSURE OF INVENTION

By the present invention a method is thus established for the preparation of a sterile dialysis fluid for medical use or a similar physiologically acceptable sterile fluid by which method water is continuously transported from a source for the same to a point of consumption, necessary concentrates in liquid and/or powder form being added during the transport, the water with our without concentrate being heated during the transport to sterilizing temperature without formation of steam and being kept at this temperature during the time required for the sterilization, whereafter it is cooled to the desired consuming temperature.

The method according to the invention is characterized in that the water heated to sterilization temperature is recirculated through the system as a whole for its sterilization by means of a recirculation line arranged substantially between the point of consumption and the said water source.

The invention also relates to a system for the preparation of a sterile dialysis fluid or a similar physiologically acceptable fluid by means of the above defined method, comprising a pipeline between the said source for the water and the point of consumption, e g a draw-off point, this pipeline comprising means for the pumping of the water, means for its heating, means for its cooling and means for the supply of necessary concentrates in liquid and/or powder form, the pipeline between the said means for heating and cooling respectively of the water being dimensioned so in relation to the capacity of the said means for pumping and heating respectively that the sojourn time of the water within this part of the pipeline will be sufficient for the sterilization.

This system is characterized by a recirculation line arranged substantially between the point of consumption and the said water source, with the help of which water heated to sterilization temperature can be recirculated through the system as a whole for its sterilization.

The said means for cooling preferably comprises a heat exchanger for a first cooling of the water with the help of the incoming, not yet heated, water. In this way good heating economy of the system is ensured.

The said means for cooling may include, if required, moreover a second heat exchanger or other cooling device for the cooling of the prepared fluid to consuming temperature. It is preferred though, to use this second heat exchanger or cooling device so as to bring the temperature down to a value just below the consuming temperature, and it is combined in such a case with a, preferably controllable, heating device for reheating the prepared fluid to the consuming temperature. The advantage of this design is that it is often simpler to control heating rather than cooling.

To obtain suitable temperatures in combination with a suitable sterilizing time for the water the pipeline from the said means for pumping of the water past the means for its heating and cooling respectively and up to a pressure reducing device is dimensioned so that it withstands the pressure which is required for maintaining the prepared fluid at the required sterilization temperature without the formation of steam.

The system in accordance with the invention can be used advantageously on pure water with addition at the same time of one or more concentrates in liquid and/or powder form for the preparation of a physilogically acceptable fluid, e g dialysis fluid or wound cleansing fluid, means being present for connecting up sources of these concentrates into the system between the point where the water is heated, and the point where the prepared fluid is cooled.

The said water source may be constituted of a water tank which is combined with means for the continuous returning of a part of the sterilized water to this tank. As a result the percentage impurity of the water in the tank is continuously reduced, enhancing thereby the safety of the subsequent sterilization being effective.

The said means for the returning of a part of the sterilized water to the tank may be constituted advantageously of a recirculation line which also comprises a heating device for heating the water to sterilizing temperature.

The said recirculation line preferably may form the primary side of a first heat exchanger which is dimensioned so that the water which passes through its secondary side is heated to the sterilization temperature at which it is to be maintained during the preparation of the said fluid.

Means are provided appropriately for dividing the water stream during the preparation of the said fluid into a main stream and in at least one partial stream, which is adapted to have the concentrate added to it before it is returned to the main stream, a conductivity meter being arranged in the main stream after the point of reunification with the respective partial stream. Alternatively, depending on the type of fluid that is being prepared, other measuring devices may be used, e g pH-meters or ion-selective electrodes for the checking of the solution prepared.

In a practical embodiment of the system in accordance with the invention the said water source is constituted of a water tank, from which a recirculation line originates with means for heating of the water to sterilizing temperature, before it is returned, and a line with means for the preparation of a physiologically acceptable fluid, e g dialysis fluid, a first and a second heat exchanger being arranged so that the water for the preparation of the said flud is heated first in the first heat exchanger to be cooled subsequently, after the preparation, in the second heat exchanger, whilst the water in the recirculation circuit is heated first in the second heat exchanger to be cooled, after further heating, in the first heat exchanger.

### BRIEF DESCRIPTION OF DRAWINGS

Fig 1 shows a system in accordance with the invention.

Fig 2 shows an alternative system for the preparation of a sterile fluid.

Fig 3, finally, shows an alternative system for the preparation of a sterile fluid.

### PREFERRED EMBODIMENTS OF THE SYSTEM ACCORDING TO THE INVENTION

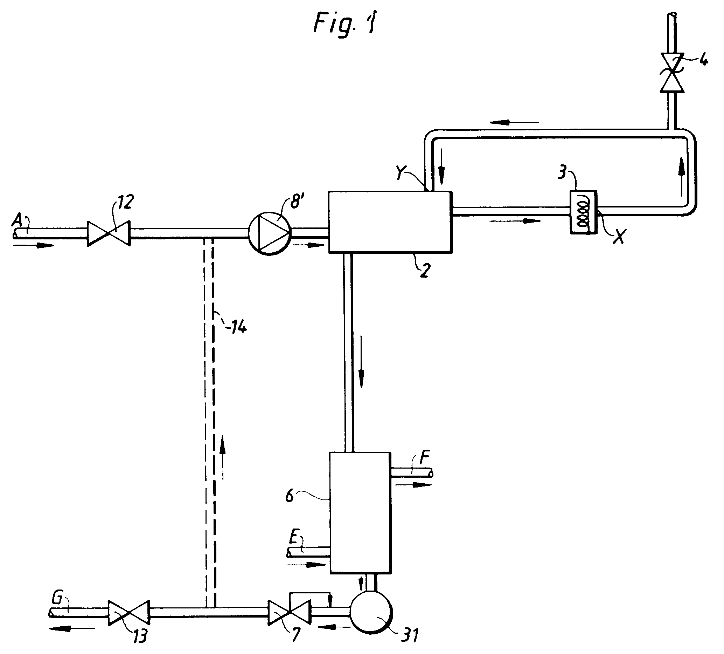

In fig 1 is shown an embodiment of the system according to the invention. The fluid is introduced at A via a valve 12 and a pump 8'. This pump is to be heat-resistant. The fluid thereafter is led through a heat exchanger 2 and a heating device 3 which forms the starting point X of a sterilization zone which terminates in point Y. Reference 4 designates a safety valve. If required the system may also be provided with a venting device. No such device, however, is shown in fig 1. The fluid thereafter passes the secondary side of the heat exchanger 2 and is conducted further to a heat exchanger 6 with an inlet E and an outlet F respectively for a cooling medium such as tap water. Here the fluid is cooled down appropriately to a temperature slightly below the utilization temperature to be heated subsequently to this temperature with the help of a heating device 31.

The fluid is then conducted via a controllable pressure reducing valve 7 to a shut-off valve 13, e g a draw-off tap or some other connection.

The sterilization zone, which starts at point X, normally terminates at point Y, but can be extended in that the valves 12 and 13 are closed and the fluid is recirculated through a recirulation line 14. In this manner the pump 8' and the components after the heat exchanger 2, including the secondary side of the latter also can be sterilized with the help of fluid heated to sterilization temperature within the system. If the valve 13 too is to be sterilized, the hot liquid can be drawn off through it during a period sufficiently long for sterility to be assured.

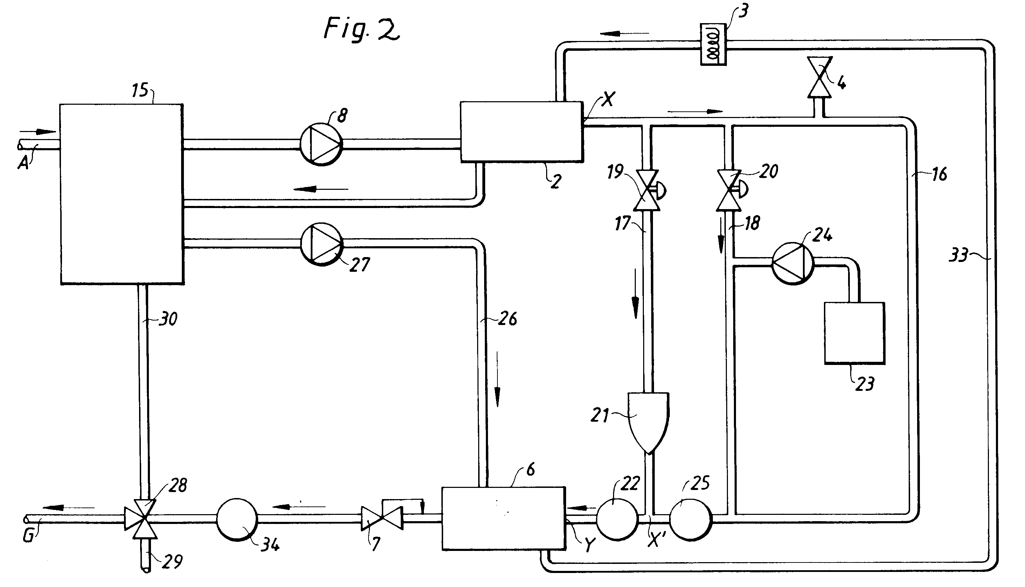

In fig 2 is shown a further system for the preparation of a sterile fluid. In this embodiment the fluid, now water, is supplied at point A, but this time to a water tank 15. With the help of a pump 8 it is then led to a heat exchanger 2 where it is heated to sterilizing temperature. The water stream is divided thereafter into a main stream 16 and at least one partial stream 17, in the case shown here into two partial streams 17 and 18. These are controlled with the help of control valves 19 and 20. To the partial stream 17 is added a salt, e g sodium bicarbonate, if a dialysis solution is to be prepared. To this end the partial stream 17 is made to pass a column 21 which contains this salt in powder form. The addition is checked subsequently, with the help of a conductivity meter 22, which appropriately is adapted so as to control the valve 19. In the same manner a second concentrate 23 may be added to the partial stream 18 with the help of a pump 24 and be checked with the help of a conductivity meter 25 which appropriately controls the control valve 20. The addition of the two concentrates can take place essentially in the manner as described in detail, for example in the US patent 4 784 495. The prepared solution is then led to a second heat exchanger 6 and via a pressure reducing valve 7 to the consuming point G.

From the tank 15 a second partial stream 26 with the help of a pump 27 is led to the heat exchanger 6 and further to a heating device 3 through a line 33. The heating device 3 and the heat exchanger 2 are dimensioned so that a sufficiently high sterilization temperature is obtained between the points X and Y during a sufficently long time whilst the liquid passes from point X to point Y. The latter time is a function of the dimensioning of the lines for the partial streams 16, 17 and 18 and the valves and other components included therein. This applies on the assumption that the concentrate used is sterile. Otherwise the point X is shifted to point X' with the result that the sterilization now has to be carried out between the points X' and Y. Reference 28 designates a three-way valve, with the help of which the fluid can be conducted either to a drain 29 or to the point of consumption G or via the line 30 back to the tank 15. The latter line is used if the system as a whole has to be sterilized, which can be done either thermally or by chemical means. The line 30 may also be used for emptying the vessel 15 to the drain 29. Reference 34, finally, designates an extra cooling device, should such a device be required at the flow and temperatures used. Alternatively 34 may designate an extra heating device if the temperature in the fluid had been reduced below the consuming temperature on passing through the heat exchanger 6.

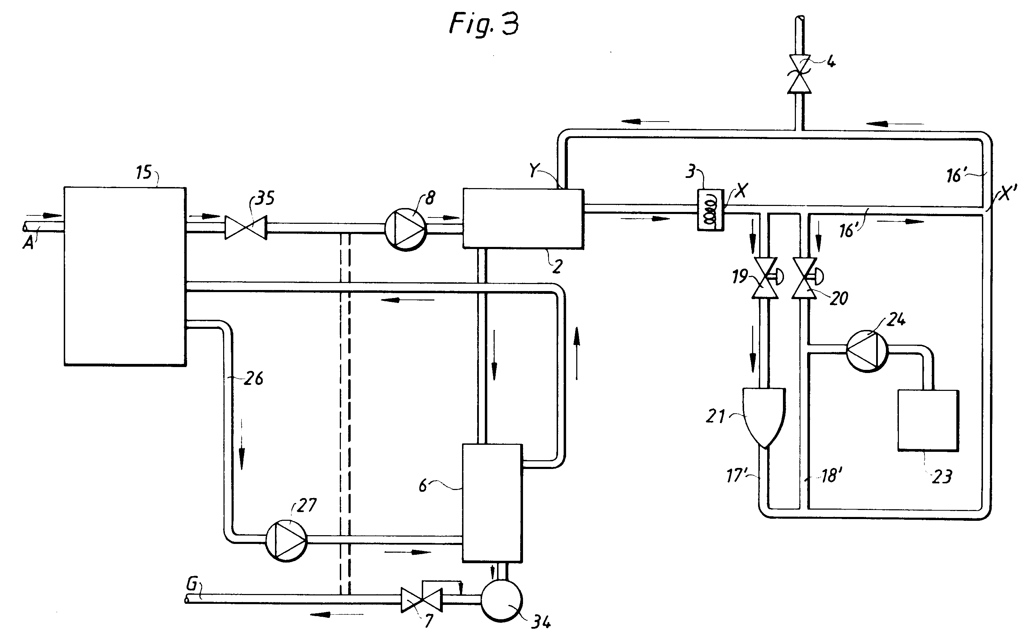

In fig 3 is shown a further system for the preparation of a sterile fluid. In this embodiment too the water is supplied at point A to a water tank 15. The water is then conducted with the help of a pump 8 via pressure control valve 35 to the heat exchanger 2. After a preheating in the heat exchanger 2 the water is taken to a heating device 3 which is situated in a main stream 16' corresponding to the main stream 16 in the system according to fig 2. Similarly the partial streams 17' and 18' with the valves 19 and 20, concentrate containers 21 and 23 and the pump 24 correspond to the partial streams 17 and 18 in fig 2 and the components provided in those streams. The flows and the concentrations in the partial streams 17' and 18' can be regulated with the help of the valves 19 and 20, if these are controlled by suitably placed conductivity meters (not shown) or other suitable checking devices. This check is facilitated if the two partial streams 17' and 18' are not joined together before reunification with the main stream 16' because it it easier to carry out the check in the more diluted main stream in the way as illustrated in fig 2 with the help of the conductivity meters 22 and 25.

After the addition of concentrates from the partial streams 17' and 18' the main stream 16' is taken via a safetly valve 4 to the secondary side of the heat exchanger 2. The sterilization takes place between the points X and Y if it is assumed that the concentrates used are sterile. Otherwise the sterilization takes place mainly between the points X' and Y. The liquid prepared is conducted subsequently via the heat exchanger 6 and a pressure reducing valve 7 to the point of consumption G. Cooling is carried out with the help of water from the tank 15 which with the help of the pump 27 is led through the heat exchanger 6 to be returned subsequently to the tank 15. If required, an additional cooling or heating may take place in a temperature regulating device 34.

Naturally, the invention is not limited simply to the embodiments described above, but may be varied within the scope of the following claims. Thus, for example, the components included may be varied within wide limits with regard to their form as well as to their function.

## Claims

1. A method for the preparation of a sterile dialysis fluid for medical use or a similar physiologically acceptable sterile fluid by which method water is continuously transported from a source (A) for the same to a point of consumption (G), necessary concentrates in liquid and/or powder form being added during the transport, the water with or without concentrate being heated during the transport to sterilizing temperature without formation of steam and being kept at this temperature during the time required for the sterilization, whereafter it is cooled to the desired consuming temperature,
**characterized** in that water heated to sterilization temperature is recirculated through the system as a whole for its sterilization by means of a recirculation line (14) arranged substantially between the point of consumption (G) and the said water source (A).

2. A method in accordance with claim 1, **characterized** in that the cooling, at least partially, takes place by means of heat exchange with incoming, not yet heated, water.

3. A method in accordance with claim 1 or 2, **characterized** in that said concentrates in liquid and/or powder form are added simultaneously with the sterilization.

4. A method in accordance with claim 1 or 2, **characterized** in that said concentrates are added in a sterile condition.

5. A system for the preparation of a sterile dialysis fluid or a similar physiologically acceptable fluid by means of the method in accordance with anyone of the claims 1-4, comprising a pipeline between the said source for the water (A) and the point of consumption (G), e g a draw-off point, this pipeline comprising means (8,8') for the pumping of the water, means (3) for its heating, means (2,6) for its cooling and means (e g 21 and 23) for the supply of necessary concentrates in liquid and/or powder form, the pipeline between the said means (3 and 2,6) for heating and cooling respectively of the water being dimensioned so in relation to the capacity of the said means (8,8' and 3) for pumping and heating respectively that the sojourn time of the water within this part (X-Y) of the pipeline will be sufficient for the sterilization, **characterized** by a recirculation line (14) arranged substantially between the point of consumption (G) and the said water source (A), with the help of which water heated to sterilization temperature can be recirculated through the system as a whole for its sterilization.

6. A system in accordance with claim 5, **characterized** in that the said means for cooling comprises a heat exchanger (2) for a first cooling of the fluid with the help of incoming, not yet heated, fluid.

7. A system in accordance with claim 6, **characterized** in that the said means for cooling comprises a second heat exchanger (6) or other cooling device for the cooling of the prepared fluid to consuming temperature.

8. A system in accordance with claim 6, **characterized** in that the said means for cooling comprises a second heat exchanger (6) or other cooling device for cooling of the water to a temperature below the consuming temperature in combination with a preferably controllable heating device (31) for the heating of the prepared fluid to consuming temperature.

9. A system in accordance with claim 5, **characterized** in that the pipeline from the said means for pumping (8,8') of the water past the means (3 and 2,6) for heating and cooling respectively of the same and up to a pressure reducing device (7) are dimensioned to withstand the pressures which are required for keeping the prepared fluid at the required sterilization temperature without the formation of steam.

10. A system in accordance with anyone of claims 5-9, **characterized** by means for the connecting up of sources for said concentrates into the system between the point (X) where the fluid is heated, and the point (Y) where the fluid is cooled.

11. A system in accordance with anyone of claims 5-10, **characterized** in that the said water source is constituted of a water tank (15), and that means (27) are provided for the continuous returning of a part of the sterilized water to this tank.

12. A system in accordance with claim 11, **characterized** in that the said means for returning of a part of the sterilized water to the tank is constituted of a recirculation line (26,33) which also contains a heating device (3) for the heating of the water to sterilizing temperature.

13. A system in accordance with claim 12, **characterized** in that the said recirculation line (33) forms the primary side for a first heat exchanger (2) which is dimensioned so that the water which is led through its secondary side, for its part too, is heated to sterilizing temperature, and is kept at this temperature during the preparation of the said fluid.

14. A system in accordance with claim 13, **characterized** by means for dividing the water stream during the preparation of the said fluid into a main stream (16) and at least one partial stream (17 and/or 18), which is adapted to have concentrate added to it before it is returned to the main stream, a conductivity meter (22,25) being arranged in the main stream after the point of reunification with the respective partial stream.

15. A system in accordance with claim 14, **characterized** in that the said source for the water is constituted of a water tank (15), from which starts out a recirculation line (26,33) with means (3) for the heating of the water to sterilizing temperature before it is returned, and a line (16) with means for the preparation of a physiologically acceptable fluid, e g dialysis fluid, a first (2) and a second (6) heat exchanger being arranged so that the water for the preparation of the said fluid is heated first in the first heat exchanger (2) to be cooled subsequently, after the preparation, in the second heat exchanger (6) whilst the water in the recirculation circuit is heated first in the second heat exchanger (6) to be cooled subsequently, after further heating, in the first heat exchanger (2).

## Patentansprüche

1. Verfahren zur Herstellung eines sterilen Dialysefluids für medizinische Verwendung oder eines ähnlichen physiologisch verträglichen sterilen Fluids, bei dem Wasser kontinuierlich von einer Quelle (A) für dieses zu einem Verbrauchspunkt (G) befördert wird, wobei erforderliche Konzentrate in Flüssigkeits- und/oder Pulverform während der Beförderung zugegeben werden, das Wasser mit oder ohne Konzentrat während der Beförderung auf Sterilisiertemperatur ohne Wasserdampfbildung erhitzt und auf dieser Temperatur während der für das Sterilisieren erforderlichen Zeit gehalten wird, wonach es auf die erwünschte Verbrauchstemperatur gekühlt wird, dadurch gekennzeichnet, daß auf Sterilisiertemperatur erhitztes Wasser durch das System als Ganzes für seine Sterilisierung mit Hilfe einer Rückführleitung (14) zurückgeführt wird, die im wesentlichen zwischen dem Verbrauchspunkt (G) und der Wasserquelle (A) angeordnet ist.

2. Verfahren nach Anspruch 1, dadurch gekennzeichnet, daß das Kühlen wenigstens teilweise mit Hilfe von Wärmeaustausch mit ankommendem, noch nicht erhitztem Wasser stattfindet.

3. Verfahren nach Anspruch 1 oder 2, dadurch gekennzeichnet, daß die Konzentrate in Flüssigkeits- und/oder Pulverform gleichzeitig mit dem Sterilisieren zugegeben werden.

4. Verfahren nach Anspruch 1 oder 2, dadurch gekennzeichnet, daß die Konzentrate in einer sterilen Form zugegeben werden.

5. System zur Herstellung eines sterilen Dialysefluids oder eines ähnlichen physiologisch verträglichen Fluids mit Hilfe des Verfahrens nach einem der Ansprüche 1 bis 4 mit einer Rohrleitung zwischen der Quelle für das Wasser (A) und dem Verbrauchspunkt (G), z.B. einem Abzugspunkt, wobei diese Rohrleitung Einrichtungen (8, 8') zum Pumpen des Wassers, Einrichtungen (3) für sein Erhitzen, Einrichtungen (2, 6) für sein Kühlen und Einrichtungen (z.B. 21 und 23) für die Zuführung erforderlicher Konzentrate in Flüssigkeits- und/oder Pulverform umfaßt und wobei die Rohrleitung zwischen den Einrichtungen (3 und 2, 6) zum Erhitzen bzw. Kühlen des Wassers in Bezug auf die Kapazität der Einrichtungen (8, 8' und 3) zum Pumpen bzw. Erhitzen derart dimensioniert sind, daß die Verweilzeit des Wassers in diesem Teil (X-Y) der Rohrleitung für das Sterilisieren ausreicht, gekennzeichnet durch eine Rückführleitung (14), die im wesentlichen zwischen dem Verbrauchspunkt (G) und der Wasserquelle (A) angeordnet ist und mit Hilfe derer auf Sterilisiertemperatur erhitztes Wasser durch das System als Ganzes für dessen Sterilisierung zurückgeführt werden kann.

6. System nach Anspruch 5, dadurch gekennzeichnet, daß die Einrichtungen zum Kühlen einen Wärmetauscher (2) für ein erstes Kühlen des Fluids mit Hilfe ankommenden, noch nicht erhitzten Fluids umfassen.

7. System nach Anspruch 6, dadurch gekennzeichnet, daß die Einrichtungen zum Kühlen einen zweiten Wärmetauscher (6) oder eine andere Kühleinrichtung zum Kühlen des vorbereiteten Fluids auf Verbrauchstemperatur umfassen.

8. System nach Anspruch 6, dadurch gekennzeichnet, daß die Einrichtungen zum Kühlen einen zweiten Wärmetauscher (6) oder eine andere Kühleinrichtung zum Kühlen des Wassers auf eine Temperatur unterhalb der Verbrauchstemperatur in Verbindung mit einer vorzugsweise steuerbaren Heizeinrichtung (31) für das Erhitzen des bereiteten Fluids auf Verbrauchstemperatur umfassen.

9. System nach Anspruch 5, dadurch gekennzeichnet, daß die Rohrleitung von den Einrichtungen zum Pumpen (8, 8') des Wassers über die Einrichtungen (3 und 2, 6) zum Erhitzen bzw. Kühlen desselben und bis zu einer Druckreduziereinrichtung (7) so dimensioniert ist, daß sie Drücken widersteht, die für das Halten des bereiteten Fluids auf der erforderlichen Sterilisiertemperatur ohne die Bildung von Wasserdampf erforderlich sind.

10. System nach einem der Ansprüche 5 bis 9, gekennzeichnet durch Einrichtungen zum Verbinden von Quellen für die Konzentrate mit dem System zwischen dem Punkt (X), wo das Fluid erhitzt wird, und dem Punkt (Y), wo das Fluid gekühlt wird.

11. System nach einem der Ansprüche 5 bis 10, dadurch gekennzeichnet, daß die Wasserquelle aus einem Wasserbehälter (15) besteht und daß Einrichtungen (27) für das kontinuierliche Rückführen eines Teils des sterilisierten Wassers zu diesem Behälter vorgesehen sind.

12. System nach Anspruch 11, dadurch gekennzeichnet, daß die Einrichtungen zum Rückführen eines Teils des sterilisierten Wassers zu dem Behälter aus einer Rückführleitung (26, 33) bestehen, die auch eine Heizeinrichtung (3) für das Erhitzen des Wassers auf Sterilisiertemperatur enthält.

13. System nach Anspruch 12, dadurch gekennzeichnet, daß die Rückführleitung (33) die Primärseite für einen ersten Wärmetauscher (2) bildet, der so dimensioniert ist, daß das Wasser, welches durch die Sekundärseite geführt wird, auch seinerseits auf Sterilisiertemperatur erhitzt und während der Bereitung des Fluids auf dieser Temperatur gehalten wird.

14. System nach Anspruch 13, gekennzeichnet durch Einrichtungen zum Teilen des Wasserstroms während der Bereitung des Fluids in einen Hauptstrom (16) und wenigstens einen Teilstrom (17 und/oder 18), welcher so ausgebildet ist, daß er vor seiner Rückkehr zu dem Hauptstrom Konzentrat zugesetzt bekommt, wobei ein Leitfähigkeitsmeßgerät (22, 25) in dem Hauptstrom nach dem Punkt der Wiedervereinigung mit dem betreffenden Teilstrom angeordnet ist.

15. System nach Anspruch 14, dadurch gekennzeichnet, daß die Quelle für das Wasser aus einem Wasserbehälter (15) besteht, von welchem eine Rückführleitung (26, 33) mit Einrichtungen (3) für das Erhitzen des Wassers auf Sterilisiertemperatur vor dessen Rückkehr und einer Leitung (16) mit Einrichtungen für die Bereitung eines physiologisch vertraglichen Fluids, z.B. von Dialysefluid, ausgeht, wobei ein erster (2) und ein zweiter (6) Wärmetauscher derart angeordnet sind, daß das Wasser für die Bereitung des Fluids zunächst in dem ersten Wärmetauscher (2) erhitzt wird, um anschließend nach der Bereitung in dem zweiten Wärmetauscher (6) gekühlt zu werden, während das Wasser in dem Rückführkreislauf zunächst in dem zweiten Wärmetauscher (6) erhitzt wird, um anschließend nach weiterem Erhitzen in dem ersten Wärmetauscher (2) gekühlt zu werden.

## Revendications

1. Méthode pour la préparation d'un fluide de dialyse stérile pour usage médical ou d'un fluide stérile physiologiquement acceptable similaire, cette méthode comprenant le transport continu d'eau d'une source (A) à un point de consommation (G), les produits concentrés nécessaires sous forme liquide et/ou de poudre étant ajoutés pendant le transport, l'eau avec ou sans produit concentré étant chauffée pendant le transport à une température de stérilisation sans formation de vapeur d'eau et étant maintenue à cette température pendant le temps nécessaire à la stérilisation, après quoi elle est refroidie à la température de consommation désirée, caractérisée en ce que l'eau chauffée à la température de stérilisation est recyclée à travers l'ensemble du système pour la stérilisation de celui-ci, au moyen d'une tuyauterie de recirculation (14) agencée sensiblement entre le point de consommation (G) et ladite source d'eau (A).

2. Méthode suivant la revendication 1, caractérisée en ce que le refroidissement est effectué, au moins partiellement, par échange de chaleur avec l'eau entrante, non encore chauffée.

3. Méthode suivant la revendication 1 ou 2, caractérisée en ce que lesdits produits concentrés sous forme liquide et/ou de poudre sont ajoutés simultanément à la stérilisation.

4. Méthode suivant la revendication 1 ou 2, caractérisée en ce que lesdits produits concentrés sont ajoutés dans un état stérile.

5. Système pour la préparation d'un fluide de dialyse stérile ou d'un fluide physiologiquement acceptable similaire par la méthode suivant une quelconque des revendications 1 à 4, comprenant une tuyauterie entre la dite source d'eau (A) et le point de consommation (G), par exemple un point de soutirage, cette tuyauterie comprenant des moyens (8,8') pour le pompage de l'eau, des moyens (3) pour son chauffage, des moyens (2,6) pour son refroidissement et des moyens (par exemple 21 et 23) pour la fourniture de produits concentrés nécessaires sous forme liquide et/ou de poudre, la tuyauterie entre lesdits moyens (3 et 2,6) pour le chauffage et le refroidissement de l'eau respectivement étant dimensionnée, par rapport à la capacité desdits moyens (8,8' et 3) pour le pompage et le chauffage respectivement, de sorte que le temps de séjour de l'eau dans cette partie (X-Y) de la tuyauterie soit suffisant pour la stérilisation, caractérisé en ce qu'il comprend une tuyauterie de recirculation (14) prévue sensiblement entre le point de consommation (G) et ladite source d'eau (A) et par laquelle l'eau chauffée à la température de stérilisation peut être recyclée à travers l'ensemble du système pour sa stérilisation.

6. Système suivant la revendication 5, caractérisé en ce que lesdits moyens de refroidissement comprennent un échangeur de chaleur (2) pour un premier refroidissement du fluide par le fluide entrant, non encore chauffé.

7. Système suivant la revendication 6, caractérisé en ce que lesdits moyens de refroidissement comprennent un deuxième échangeur de chaleur (6) ou un autre dispositif de refroidissement pour le refroidissement du fluide préparé à la température de consommation.

8. Système suivant la revendication 6, caractérisé en ce que lesdits moyens de refroidissement comprennent un deuxième échangeur de chaleur (6) ou un autre dispositif de refroidissement pour refroidir l'eau à une température inférieure à la température de consommation, en combinaison avec un dispositif de chauffage de préférence réglable (31) pour le chauffage du fluide préparé à la température de consommation.

9. Système suivant la revendication 5, caractérisé en ce que la tuyauterie partant desdits moyens de pompage (8,8') de l'eau, passant dans les moyens (3 et 2,6) de chauffage et de refroidissement de l'eau respectivement, et aboutissant à un dispositif de réduction de pression (7) est dimensionnée de manière à résister aux pressions qui sont requises pour maintenir le fluide préparé à la température de stérilisation requise sans formation de vapeur.

10. Système suivant une quelconque des revendications 5 à 9, caractérisé en ce qu'il comprend des moyens pour le raccordement de sources de dits produits concentres dans le système entre le point(X) où le fluide est chauffé et le point (Y) où le fluide est refroidi.

11. Système suivant une quelconque des revendications 5 à 10, caractérisé en ce que ladite source d'eau est constituée d'une cuve d'eau (15) et en ce que des moyens (27) sont prévus pour le retour continu d'une partie de l'eau stérilisée vers cette cuve.

12. Système suivant la revendication 11, caractérisé en ce que lesdits moyens de retour d'une partie de l'eau stérilisée à la cuve comprennent une tuyauterie de recirculation (26,33) qui contient également un dispositif de chauffage (3) pour le chauffage de l'eau à la température de stérilisation.

13. Système suivant la revendication 12, caractérisé en ce que ladite tuyauterie de recirculation (33) forme le côté primaire d'un premier échangeur de chaleur (2) qui est dimensionné de sorte que l'eau qui circule dans son côté secondaire, pour sa part également, est chauffée à la température de stérilisation et est maintenue à cette température pendant la préparation dudit fluide.

14. Système suivant la revendication 13, caractérisé en ce qu'il comprend des moyens de division du flux d'eau, pendant la préparation dudit fluide, en un flux principal (16) et au moins un flux partiel (17 et/ou 18), qui permet d'y ajouter un produit concentré avant son retour au flux principal, un conductivimètre (22,25) étant prévu dans le flux principal après le point de réunification avec le flux partiel respectif.

15. Système suivant la revendication 14, caractérisé en ce que ladite source d'eau est constituée d'une cuve d'eau (15), d'où part une tuyauterie de recirculation (26,33) comportant des moyens (3) pour le chauffage de l'eau à la température de stérilisation avant son retour, et une tuyauterie (16) comportant des moyens pour la préparation d'un fluide physiologiquement acceptable, par exemple un fluide de dialyse, un premier (2) et un deuxième (6) échangeurs de chaleur étant agencés de sorte que l'eau pour la préparation dudit fluide est chauffée d'abord dans le premier échangeur de chaleur (2) pour être ensuite refroidie, après la préparation, dans le deuxième échangeur de chaleur (6) tandis que l'eau dans le circuit de recirculation est chauffée d'abord dans le deuxième échangeur de chaleur (6) pour être ensuite refroidie, après nouveau chauffage,dans le premier échangeur de chaleur (2).
